**Europäisches Patentamt**

**European Patent Office**

**Office européen des brevets**

(19)

(11) Veröffentlichungsnummer: **0 432 591 A1**

## (12) EUROPÄISCHE PATENTANMELDUNG

(21) Anmeldenummer: 90122988.0

(22) Anmeldetag: 30.11.90

(51) Int. Cl.5 **A61M 5/44**

(30) Priorität: 07.12.89 DE 3940519

(43) Veröffentlichungstag der Anmeldung:
**19.06.91 Patentblatt 91/25**

(84) Benannte Vertragsstaaten:
**AT BE CH DE DK FR GB IT LI LU NL SE**

(71) Anmelder: **TRANSMED Medizintechnik GmbH**
**Josefstrasse 4**
**W-4798 Wünnenberg 2(DE)**

(72) Erfinder: **Alt, Erwin**
**Am Anger 6**
**W-8098 Pfaffing(DE)**
Erfinder: **Rappert, Klaus-Jürgen**
**Josefstrasse 4**
**W-4798 Wünnenberg 2(DE)**

(74) Vertreter: **Kehl, Günther, Dipl.-Phys. et al**
**Patentanwälte Hagemann & Kehl Ismaninger**
**Strasse 108 Postfach 86 03 29**
**W-8000 München 86(DE)**

(54) **Aufwärm- und Auftaugerät.**

(57) Aufwärm- und Auftaugerät für medizinische Infusions- und Transfusionslösungen, mit einem Wasserbadbehälter (2) für die aufzuwärmenden/aufzutauenden in Behältnissen befindlichen Lösungen, mit einer temperaturgeregelten, von einer Steuereinheit angesteuerten Heizeinrichtung für das Wasserbad und mit einem eine Umwälzpumpe enthaltenden Umwälzkreislauf zur Umwälzung des Wasserbades.

Zur leichteren Handhabung des Geräts ist der Wasserbadbehälter (2) lösbar mit dem übrigen Auftau- und Aufwärmgerät verbunden.

EP 0 432 591 A1

# AUFWÄRM- UND AUFTAUGERÄT

Die Erfindung bezieht sich auf ein Aufwärm- und Auftaugerät für medizinische Infusions- und Transfusionslösungen, mit einem Wasserbadbehälter für die aufzuwärmenden/aufzutauenden in Behältnissen befindlichen Lösungen, mit einer temperaturgeregelten, von einer Steuereinheit angesteuerten Heizeinrichtung für das Wasserbad und mit einem eine Umwälzpumpe enthaltenden Umwälzkreislauf zur Umwälzung des Wasserbades.

Tiefgefrorene Infusions- und Transfusionslösungen, wie beispielsweise gefrorenes Blutplasma, müssen schnell und schonend aufgetaut werden, um im Notfall oder bei einer Operation zur Verfügung zu stehen. Je schneller der Auftauvorgang durchgeführt werden kann, umso kleiner kann der Vorrat an im aufgetauten Zustand bereitgehaltenen Lösungen gehalten werden.

Es sind daher bereits Vorrichtungen bekannt geworden, mit denen tiefgefrorene Lösungen mittels Mikrowellen aufgetaut und/oder auf Temperatur angewärmt werden können. Solche Geräte weisen jedoch die Gefahr auf, daß sich infolge stehender Wellen in dem Mikrowellenresonator Stellen mit zu hoher Temperatur bilden, an denen beim Auftauen von Blutkonserven Bestandteile des Bluts geschädigt werden können. Außerdem sind Mikrowellengeräte aufwendig und teuer.

Auch Aufwärm- und Auftaugeräte mit Wasserbadbehältern, bei denen die Behältnisse mit den aufzutauenden Lösungen in ein erwärmtes Wasserbad eingehängt werden, sind bekannt. Um eine ausreichende Wärmezufuhr von dem Wasserbad zu den Behältnissen zu gewährleisten und um zu verhindern, daß die Behältnisse von abgekühltem Wasser umgeben werden, ist vorgesehen, daß die Behältnisse an einem hin- und herschwingenden Gestell aufgehängt werden. Ein solches mechanisch angetriebenes Gestell ist jedoch in der Herstellung aufwendig und teuer, störanfällig und schwierig zu reinigen. Falls die Schwingung zu heftig ausgeführt wird, können dadurch - beim Aufwärmen von Blutkonserven - empfindliche Bestandteile des Bluts, wie beispielsweise Erythrozyten, geschädigt werden.

Ein Aufwärm- und Auftaugerät der eingangs genannten Art ist aus den Unterlagen des deutschen Gebrauchsmusters G 88 09 120.1 bekannt. Bei dem bekannten Gerät besteht die Steuereinheit und der Wasserbadbehälter aus einer fest verbundenen Einheit. In der Praxis hat es sich gezeigt, daß es häufig erforderlich ist, den Wasserbadbehälter gründlich zu reinigen. Es ist bei dem bekannten Gerät wegen der erwähnten einstückigen Bauweise schwierig, zumal in dem Wasserbadbehälter auch noch die Heizeinrichtung angeordnet ist.

Der Erfindung liegt daher die Aufgabe zugrunde ein Auftaugerät der eingangs genannten Art zu schaffen, das unter Beibehaltung seiner Vorteile des schonenden und schnellen Aufwärmens und Auftauens, seiner einfachen und kostengünstigen Herstellung und seiner leichten Handhabbarkeit vor allem leicht zu reinigen und zu warten ist und somit einen unter hygienischen Gesichtspunkten sicheren Betrieb gewährleistet.

Die Lösung dieser Aufgabe ist dadurch erreicht, daß der Wasserbadbehälter lösbar mit dem übrigen Aufwärm- und Auftaugerät verbunden ist.

Durch die Lösbarkeit des Wasserbadbehälters kann dieser leicht zu Reinigungszwecken entnommen und beispielsweise in einem Spülbecken gründlich gereinigt und desinfiziert werden, so daß die medizinischen Hygieneanforderungen beim Betrieb des Gerätes erfüllt sind.

Nach einer vorteilhaften Ausführungsform der Erfindung weist das Gerät einen in den Wasserbadbehälter eintauchbaren Taucharm auf, der mindestens eine mit der Saugseite der Umwälzpumpe verbundene Ansaugöffnung und mindestens eine mit der Druckseite der Umwälzpumpe verbundene Ausströmöffnung aufweist. In diesem Fall kann der Wasserbadbehälter völlig frei van den Anschlüssen für die Ansaug- und Ausströmöffnungen gehalten werden, wodurch die Reinigung des weiteren erleichtert ist. Die Ansaugöffnung befindet sich vorteilhafterweise am unteren Ende des Taucharmes, also in der Nähe des Bodens des Wasserbadbehälters, so daß der Wasserbadbehälter gewünschtenfalls auch über die Ansaugöffnung entleert werden kann.

Nach einer weiteren vorteilhaften Ausführungsform der Erfindung ist vorgesehen, daß der Taucharm um eine oberhalb des Wasserbadbehälters liegende Achse verschwenkbar ist. Dank dieser Bauweise kann er einfach aus dem Wasserbadbehälter herausgeschwenkt und der Wasserbadbehälter, insbesondere zu Reinigungszwecken, entfernt werden.

An dem Taucharm sind nach einer weiteren vorteilhaften Ausführungsform mehrere Ausströmöffnungen vorgesehen, die auf die im Wasserbad angeordneten die Infusions-/Transfusionslösungen enthaltenden Behältnisse gerichtet sind. Das aus den Ausströmöffnungen ausströmende, im Kreislauf geführte Wasser strömt einerseits die Behältnisse an, so daß sich keine Bereiche kalten Wassers um die Behältnisse herum bilden. Zum andern übt auch die auf die Behältnisse gerichtete Strömung, insbesondere, wenn es sich bei den Behältnissen um Beutel handelt, eine leichte mechanische Wirkung auf diese aus, so daß der Inhalt der Behältnisse etwas durchmischt wird, wodurch die Wärmeübertragung von dem Wasserbad auf die Behältnisse unterstützt wird.

Nach einer weiteren vorteilhaften Ausführungsform sind an dem Taucharm auch zwei mit einer Steuereinheit verbundene Füllstandsfühler angeordnet. Ein Füllstandsfühler, der im Bereich des Wasserspiegels bei gefülltem Wasserbadbehälter angeordnet ist, schafft die Möglichkeit, den Wasserstand entweder automatisch oder halbautomatisch, d.h. durch Alarmgabe bei einem zu niedrigen Wasserstand, zu regeln. Durch die erfindungsgemäße Bauweise, bei der die Füllstandsfühler an dem Taucharm angeordnet sind, wird der Behälter von diesen Aggregaten völlig freigehalten, so daß diese bei einer Reinigung des Behälters nicht stören. Die Anordnung eines Füllstandfühlers in der Nähe des Bodens des Wasserbadbehälters ermöglicht auch ein automatisches Entleeren des Wasserbadbehälters, wobei der Entleervorgang durch Abschalten einer Entleerungspumpe oder durch Schließen eines Entleerungsventils dann beendet ist, wenn der in der Nähe des Behälterbodens angeordnete Füllstandsfühler meldet, daß der Wasserspiegel bis dorthin abgefallen ist.

An dem Taucharm kann auch gemäß einer weiteren Ausführungsform Temperaturfühler angeordnet werden, die an die Steuereinheit zum Zwecke der Übertragung eines Meßwertes angeschlossen sind. Der Meßwert kann zur Anzeige oder auch zur Ansteuerung einer Heizeinrichtung verwendet werden. Die Heizeinrichtung ist nach einer weiteren Ausführungsform als mindestens ein Durchlauferhitzer ausgebildet. Sie kann dabei außerhalb des Wasserbadbehälters in dem Umwälzkreislauf angeordnet werden.

Vorzugsweise weist die Heizeinrichtung mehrere Leistungsstufen auf und die Steuereinheit enthält eine die Differenz zwischen einem vorgegebenen Temperatursollwert und dem durch den Temperaturfühler ermittelten Temperaturistwert bildende Schaltung, so daß die Leistungsstufe der Heizeinrichtung entsprechend der Größe der ermittelten Differenz durch die Steuereinheit angesteuert wird. Das bedeutet, daß im Falle einer großen Abweichung zwischen dem Istwert und dem Temperatursollwert (meist 36°G) die höchste Leistungsstufe der Heizeinrichtung abgerufen wird, während bei nur kleinen Abweichungen, beispielsweise von einem Grad, nur die niedrigste Leistungsstufe der Heizeinrichtung angesteuert wird. In der Praxis hat sich die Verwendung von drei Leistungsstufen als ausreichend zu einer sicheren und effektiven Temperaturregelung erwiesen.

Zur Sicherstellung der medizinischen Hygiene beim Betrieb hat es sich als besonders vorteilhaft erwiesen, eine Entkeimungskammer in dem Umwälzkreislauf anzubringen. Eine solche Entkeimungskammer besteht nach einer weiteren vorteilhaften Ausführungsform aus einem UV-Strahler, über den das umgewälzte Wasser geführt wird.

Vorzugsweise weist das Gerät einen über die Steuereinrichtung ansteuer baren mit einem Wasserhahn verbindbaren Wasserzulauf und einen über die Steuereinrichtung ansteuerbaren Wasserablauf auf. Die oben erwähnten Füllstandsfühler können bei dieser Ausführungsform zur automatischen Niveauregulierung des Wasserspiegels verwendet werden. Das bedeutet, bei Verdunstung oder sonstigem Wasserverlust, vermittelt der obere Füllstandsfühler ein Signal an die Steuereinheit, die dann den Wasserzulauf, beispielsweise durch Öffnen eines Magnetventils, ansteuert, so daß Wasser aus der Wasserleitung solange nachfließt, bis der Sollfüllstand wieder erreicht ist. Durch Eingabe eines entsprechenden Steuerbefehls an die Steuereinrichtung kann der Wasserbehälter automatisch geleert werden, wobei nach erfolgter Leerung der untere Füllstandanzeiger ein entsprechendes Signal liefert, um den Entleerungsvorgang zu beenden, beispielsweise durch Abschalten einer Entleerungspumpe oder durch Schließen eines Entleerungsventils. Auch das erneute Befüllen des Wasserbadbehälters kann somit auf einfache Weise, gewissermaßen auf Knopfdruck erfolgen.

Die Erfindung wird im folgenden anhand des in den Figuren schematisch dargestellten Ausführungsbeispiels näher erläutert. Es zeigt:

Figur 1:     ein Aufwärm- und Auftaugerät gemäß der Erfindung in Frontansicht;

Figur 2:     das Aufwärm- und Auftaugerät wie in Figur 1, jedoch mit hochgeschwenktem Taucharm;

Figur 3:     das Aufwärm- und Auftaugerät gemäß den Figuren 1 und 2, jedoch von der Seite gesehen;

Figur 4:     den Deckel für den Wasserbadbehälter in Draufsicht;

Figur 5:     einen Einsatz zur Aufnahme eines Behältnisses mit Auftaugut;

Figur 6:     den Einsatz gemäß Figur 5 von der Seite gesehen;

Figur 7:     den Umwälzkreislauf;

Figur 8:     ein Schaltschema.

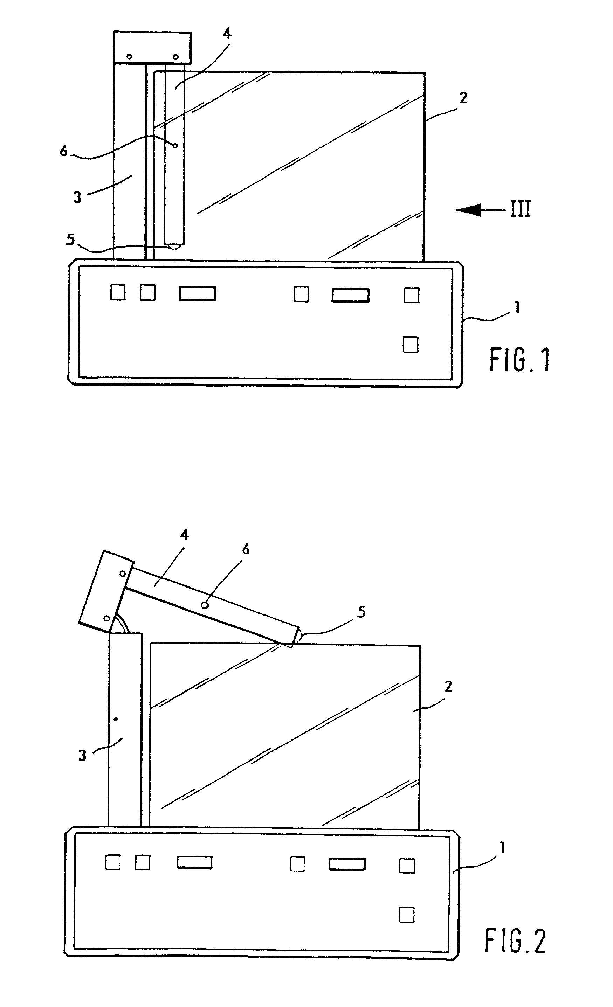

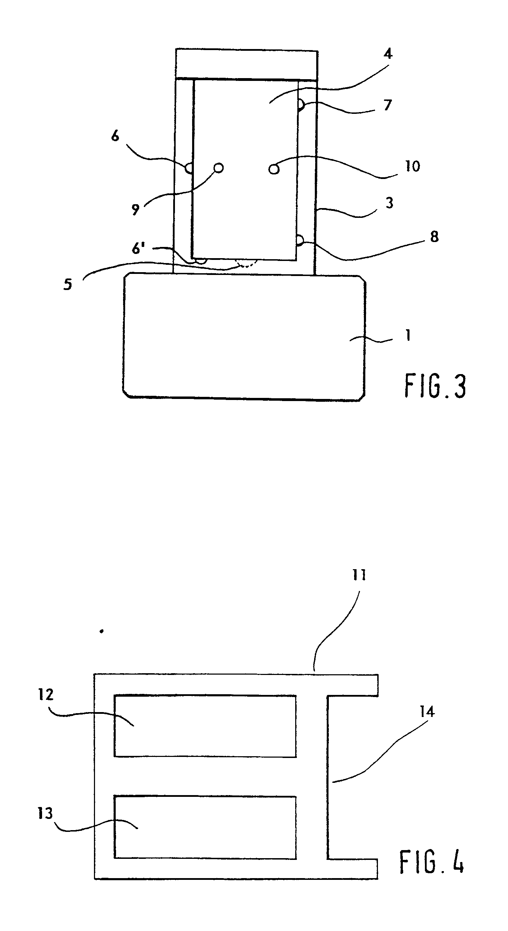

Das in den Figuren 1 und 2 gezeigte Gerät besteht aus einem Grundkörper 1, der einen Durchlauferhitzer, einen UV-Strahler als Entkeimungskammer, eine Umlaufpumpe, mehrere Magnetventile und eine Steuereinheit enthält, was weiter unten beschrieben werden soll. Auf dem Grundkörper 1 steht ein Wasserbadbehälter 2, der aus einer oben offenen, jedoch ansonsten vollkommen geschlossenen, von Anschlüssen und Durchbrüchen freien Wanne besteht. An dem Grundkörper 1 ist eine Säule 3 befestigt, die einen in den Wasserbadbehälter 2 eintauchenden Taucharm 4 trägt. Am unteren Ende des Taucharms befindet sich eine durch ein Auslaufsieb abgedeckte Ansaugöffnung 5. An der Seite, etwa in halber Höhe, befindet sich ein Temperaturfühler 6. An der Unterkante des Taucharms 4 ist ein weiterer Sicherheitstemperaturfühler 6' angebracht (in Figur 1 nicht zu erkennen). An der abgewandten Seite des Taucharms sind zwei Füllstandsfühler angeordnet, von denen der eine nahezu am unteren Ende des Taucharms und der andere etwa im Bereich des Wasserspiegels bei gefülltem Wasserbadbehälter angeordnet ist (beide Füllstandsfühler sind bei der Darstellung in Figur 1 nicht zu erkennen).

Um den Wasserbadbehälter 2, insbesondere zu Reinigungszwecken, abnehmen zu können, kann der Taucharm 4 nach oben geschwenkt werden. Diese Position ist in Figur 2 dargestellt. Etwas oberhalb der in Figur 2 gezeigten Position kann der Taucharm 4 eingerastet werden, so daß der Wasserbadbehälter 2 bequem entnommen werden kann. Beim Hochklappen des Taucharms 4 in die in Figur 2 gezeigte Position wird des weiteren ein Positionsfühler (nicht dargestellt) betätigt, der die Umwälzpumpe sperrt, so daß in dieser Position des Taucharms kein Wasser aus diesem austreten kann.

In Figur 3 ist das Gerät in einer Seitenansicht (ohne Wasserbadbehälter) gezeigt. Es ist insbesondere der Grundkörper 1, die auf dem Grundkörper 1 angeordnete Säule 3 und der Taucharm 4 zu erkennen, der im wesentlichen aus einer rechteckigen Platte besteht. An dem Taucharm sind die bereits beschriebenen Temperaturfühler 6, 6' und Füllstandsfühler 7 und 8 angeordnet. Etwa in halber Höhe des Taucharms befinden sich zwei Auslaßdüsen 9, 10, deren Wasserstrahl beim Betrieb des Gerätes auf die das aufzuwärmende oder aufzutauende Gut enthaltenden Behältnisse gerichtet ist.

Beim Betrieb des Gerätes wird der Wasserbadbehälter durch einen Deckel 11 abgedeckt, wie er in Figur 4 gezeigt ist. Der Deckel weist zwei Ausnehmungen 12, 13 für zwei weiter unten zu beschreibende Einsätze auf. Eine weitere randseitige Ausnehmung 14 ist für den Taucharm vorgesehen.

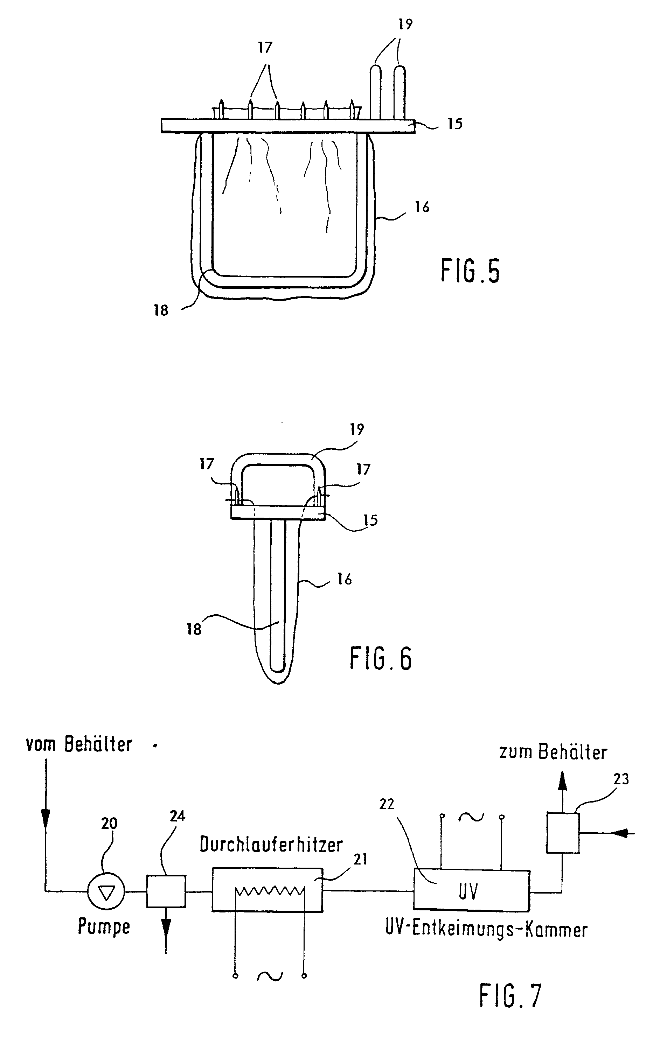

Ein Einsatzteil ist in den Figuren 5 und 6 dargestellt. Es besteht aus einer länglichen rechteckigen Grundplatte 15, die eine solche Größe hat, daß sie eine Ausnehmung 12 oder 13 des in Figur 4 gezeigten Deckels 11 überdeckt. Die Grundplatte 15 weist eine sich über einen Großteil ihrer Länge erstreckende Längsöffnung auf, in die eine sackförmige Kunststoffolie 16 eingehängt werden kann. Die Ränder der sackförmigen Kunststoffolie 16 sind mithilfe von Befestigungszähnen 17 festgehalten, die am Rand der Längsöffnung angeordnet sind. Diese können noch mit einer Halteleiste (nicht dargestellt) überdeckt werden.

Durch einen U-förmigen Versteifungsbügel 18, wird sichergestellt, daß die sackförmige Kunststofolie 16 ihre Form behält und beim Eintauchen in das Wasserbad nicht nach oben schwimmt. Zwei Haltegriffe 19 an der Oberseite des Einsatzes erleichtern dessen Handhabung.

Zur Erzeugung eines Wasserumlaufes in der Aufwärm- und Auftauvorrichtung ist ein Umwälzkreislauf vorgesehen, der in Figur 7 dargestellt ist. Dieser beginnt an der Ansaugöffnung 5 und führt über einen durch den Taucharm 4 geführten Schlauch zu der im Grundkörper 1 angeordneten Umwälzpumpe 20, von dort über einen dreistufigen Durchlauferhitzer 21 zu einer UV-Entkeimungskammer 22. Die UV-Entkeimungskammer 22 enthält einen Quecksilberniederdruckbrenner, der Strahlung mit einer Wellenlänge von 253,7 nm erzeugt. Von der Entkeimungskammer 22 führt eine weitere Schlauchleitung über das Innere des Taucharmes zu den Auslaßdüsen 9 und 10 (siehe Figur 3). An den Umwälzkreislauf sind ein steuerbarer Wasserzulauf 23 und ein steuerbarer Wasserablauf 24 angeschlossen.

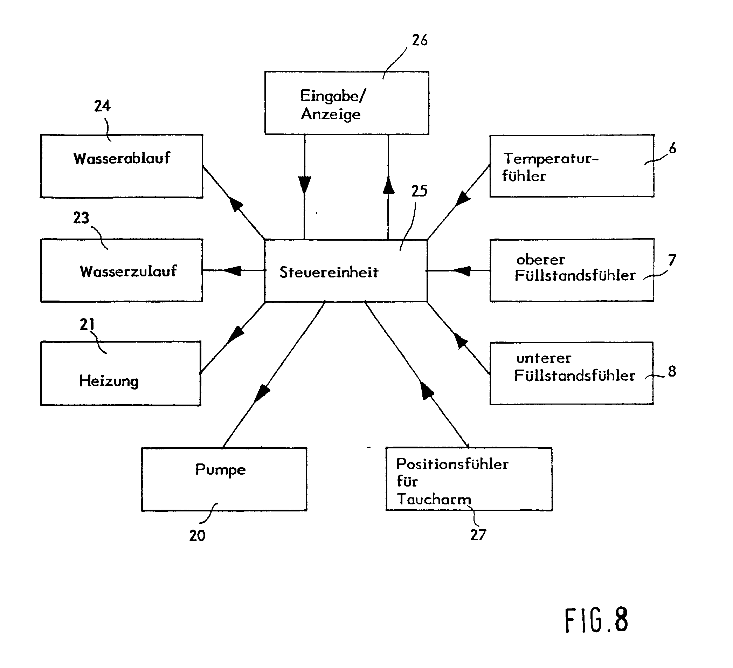

Die Verbindung der einzelnen Komponenten mit der Steuereinheit 25 ist in Figur 8 gezeigt. Wie zu erkennen, ist die Steuereinheit 25 mit einem Eingabe- und Anzeigefeld 26 verbunden. Außer von dem Eingabefeld erhält die Steuereinheit 25 Signale von den Temperaturfühlern 6, 6' den Füllstandsfühler 7 und 8 und dem Positionsfühler für den Taucharm 4. Aus den genannten Eingabesignalen leitet die Steuereinheit Steuersignale für den Betrieb der Pumpe, für den Betrieb der dreistufigen Heizung sowie für den Wasserzulauf und den Wasserablauf ab.

Nachfolgend wird der Betrieb des Geräts beschrieben:

Das Gerät ist über eine Schlauchleitung an einen (geöffneten) Wasserhahn sowie über eine weitere Schlauchleitung an einen Ausguß angeschlossen. Wenn der Wasserbadbehälter 2, wie in Figur 2 gezeigt, auf dem Grundkörper 1 angeordnet ist, wird durch Betätigung einer Drucktaste an der Frontseite des Grundkörpers 1 das Befüllen des Wasserbadbehälters 2 eingeleitet. Hierzu wird von der Steuereinheit an den Wasserzulauf 23 ein Signal gegeben, das ein Magnetventil öffnet, so daß über den Wasserzulauf 23 (Figur 7) frisches Leitungswasser in den Wasserbehälter 2 einströmt. Sobald der obere Füllstandsfühler 7 mit dem Wasserspiegel in Berührung kommt, sendet er ein entsprechendes Signal an die Steuereinheit 25, wodurch der Wasserzulauf gesperrt wird. Der Temperaturfühler 6 meldet an die Steuereinheit die tatsächliche Temperatur des in den Wasserbehälter eingefüllten Frischwassers von beispielsweise 10° C. Diese Temperaturmessung wird von dem zweiten, unabhängigen Temperaturfühler 6' überprüft. Die Steuereinheit errechnet die Differenz zur Solltemperatur von 36°C und sendet ein Steuersignal an die Heizung 21, durch das diese zum Betrieb auf der höchsten Leistungsstufe veranlaßt wird, um ein möglichst rasches Aufwärmen des Wasserbades zu bewirken. Bei Erreichen von 34°C wird durch Umschalten auf die zweite Heizstufe die Heizleistung etwa halbiert, wonach eine weitere Halbierung der Heizleistung bei 35°C erfolgt. Sobald einer der Temperaturfühler 6, 6' eine Temperatur von 36°C meldet, wird die Heizung völlig abgeschaltet. Während des gesamten Vorgangs ist die Umwälzpumpe intermittierend in Betrieb. Das umgewälzte Wasser wird ständig in der UV-Entkeimungskammer keimfrei gehalten.

Nach Erreichen der Solltemperatur, oder auch schon früher, wird der in Figur 4 gezeigte Deckel auf das Wasserbad mit zwei in Figur 5 gezeigten Einsätzen gelegt. In die Einsätze werden Beutel mit dem aufzutauenden Gut eingesetzt, wobei sich die Kunststoffolie 16 dicht und luftfrei an die Wandung des Beutels anlegt. Die aus den Auslaßdüsen 9, 10 austretende Warmwasserströmung gewährleistet eine homogene Temperatur im Wasserbad und damit ein rasches und doch schonendes Aufwärmen der kühlen oder gefrorenen Lösungen.

An der Frontseite des Grundkörpers befindet sich eine Zeitanzeige mit START/STOP-Taste, nach der die für die jeweilige Lösung erforderliche Auftau- und Anwärmzeit gemessen werden kann.

Falls nach mehreren Betriebstagen eine Reinigung des Wasserbadbehälters 2 erforderlich ist, wird an der Frontseite des Grundkörpers 1 eine Taste betätigt, die das Entleeren bewirkt, worauf die Steuereinheit 25 den Wasserablauf 24 ansteuert und gleichzeitig die Heizung 21 außer Betrieb setzt. Sobald der untere Füllstandsfühler 8 meldet, daß der Wasserpegel bis zu diesem abgefallen ist, wird das Gerät abgeschaltet und der Wasserbadbehälter 2 kann nach Hochklappen des Taucharmes 4 (wie in Figur 2 gezeigt) zu Reinigungszwekken entnommen werden.

**Ansprüche**

1. Aufwärm- und Auftaugerät für medizinische Infusions- und Transfusionslösungen, mit einem Wasserbadbehälter (2) für die aufzuwärmenden/aufzutauenden in Behältnissen befindlichen Lösungen, mit einer temperaturgeregelten von einer Steuereinheit (25) angesteuerten Heizeinrichtung (21) für das Wasserbad und mit einem eine Umwälzpumpe (20) enthaltenden Umwälzkreislauf zur Umwälzung des Wasserbades, dadurch **gekennzeichnet,** daß der Wasserbadbehälter (2) lösbar mit dem übrigen Auftau- und Aufwärmgerät verbunden ist.

2. Gerät nach Anspruch 1, gekennzeichnet durch einen von oben in den Wasserbadbehälter (2) eintauchbaren Taucharm (4), der mindestens eine mit der Saugseite der Umwälzpumpe (20) verbundene Ansaugöffnung (5) und mindestens eine mit der Druckseite der Umwälzpumpe (20) verbundene Ausströmöffnung (9, 10) aufweist.

3. Gerät nach Anspruch 2 oder 3, dadurch gekennzeichnet, daß der Taucharm (4) um eine oberhalb des Wasserbadbehälters (2) liegende Achse verschwenkbar ist.

4. Gerät nach Anspruch 2 oder 3, dadurch gekennzeichnet, daß an dem Taucharm (4) mehrere Ausströmöffnungen (9, 10) vorgesehen sind, die auf die im Wasserbad angeordneten Behältnisse gerichtet sind.

5. Gerät nach einem der Ansprüche 2 bis 4, dadurch gekennzeichnet, daß an dem Taucharm (4) zwei an die Steuereinheit angeschlossene Füllstandsfühler (7, 8) angeordnet sind.

6. Gerät nach einem der Ansprüche 2 bis 5, dadurch gekennzeichnet, daß an dem Taucharm (4) mindestens ein an die Steuereinheit angeschlossener Temperaturfühler (6, 6') angeordnet ist.

7. Gerät nach einem der Ansprüche 1 bis 6, dadurch gekennzeichnet, daß die Heizeinrichtung als mindestens ein Durchlauferhitzer (21) ausgebildet ist.

8. Gerät nach den Ansprüchen 6 und 7, dadurch gekennzeichnet, daß die Heizeinrichtung (21) mehrere Leistungsstufen aufweist, daß die Steuereinheit (25) eine die Differenz zwischen einem vorgegebenen Temperatursollwert und dem durch den Temperaturfühler ermittelten Temperaturistwert bildende Schaltung aufweist, und daß die Leistungsstufe der Heizeinrichtung (21) entsprechend der Größe der Differenz durch die Steuereinheit (25) angesteuert wird.

9. Gerät nach einem der Ansprüche 1 bis 8, dadurch gekennzeichnet, daß in dem Umwälzkreislauf eine Entkeimungskammer (22) angeordnet ist.

10. Gerät nach Anspruch 9, dadurch gekennzeichnet, daß die Entkeimungskammer (22) einen UV-Strahler enthält.

11. Gerät nach einem der Ansprüche 1 bis 10, dadurch gekennzeichnet, daß es einen über die Steuereinrichtung (25) ansteuerbaren, mit einem Wasserhahn verbindbaren Wasserzulauf (23) und einen über die Steuereinrichtung ansteuerbaren Wasserablauf (24) aufweist.

FIG.1

FIG.2

FIG.3

FIG.4

FIG.5

FIG.6

vom Behälter

zum Behälter

Pumpe

Durchlauferhitzer

UV

UV-Entkeimungs-Kammer

FIG.7

9

FIG.8

# Europäisches Patentamt

## EUROPÄISCHER RECHERCHENBERICHT

Nummer der Anmeldung

**EP 90 12 2988**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | DE-U-8 809 120   (TRANSMED MEDIZINTECHNIK VER-TRIEBS GmbH)<br>* Insgesamt * | 1 | A 61 M 5.44 |
| A | EP-A-0 273 201   (NICOLAS MARCHIANI CHATELAIN)<br>* Fig.; Zusamenfassung * | 1 | |
| A | US-A-4 486 389   (DARNELL et al.)<br>* Figuren 1,2,4; Spalte 1, Zeile 62 - Spalte 2, Zeile 15 * | 1 | |
| A | EP-A-0 318 924   (BARKEY)<br>* Figuren 1,2; Spalte 1, Zeile 39 - Spalte 2, Zeile 12; Spalte 3, Zeile 46 - Spalte 4, Zeile 4 * | 1 | |

| | RECHERCHIERTE SACHGEBIETE (Int. Cl.5) |
|---|---|
| | A 61 M<br>A 61 K |

Der vorliegende Recherchenbericht wurde für alle Patentansprüche erstellt

| Recherchenort | Abschlußdatum der Recherche | Prüfer |
|---|---|---|
| Den Haag | 15 März 91 | SEDY. R. |